# EUROPEAN PATENT SPECIFICATION

(11) **EP 3 910 008 B1**
(45) Date of publication and mention of the grant of the patent: **02.09.2026**
(21) Application number: 20739181.4
(22) Date of filing: 13.01.2020
(51) Int. Cl.: C08J 3/28, A23L 33/10, A61K 8/02, A61K 8/67, A61K 8/73, A61K 47/69, A61K 49/00, A61K 51/06, A61K 51/12, A61P 35/00, A61Q 19/00, A61Q 19/02, A61Q 19/08, C08B 37/08, C08J 3/14, C08L 5/08

(54) **METHOD FOR SYNTHESIZING HYALURONIC ACID NANOPARTICLES**
VERFAHREN ZUR HERSTELLUNG VON HYALURONSÄURE-NANOPARTIKELN
PROCÉDÉ DE SYNTHÈSE DE NANOPARTICULES D'ACIDE HYALURONIQUE

(30) Priority: 11.01.2019 KR 20190003966
(43) Date of publication of application: 17.11.2021
(73) Proprietor: Kyungpook National University Industry-Academic Cooperation Foundation, Daegu 41566 (KR)
(72) Inventor: YOO, Jeong Soo, Daegu 41944 (KR); LEE, Woong Hee, Daegu 41944 (KR); PARK, Wonchoul, Seoul 01799 (KR)
(74) Representative: Richardt Patentanwälte PartG mbB
(86) International application number: PCT/KR2020/000612
(87) International publication number: WO 2020/145790

(56) References cited:
- KR-A- 20060 008 913
- KR-A- 20170 087 427
- US-A1- 2012 283 428
- US-A1- 2018 327 554
- PARK DONGJEONG: "Conjugation of hyaluronic acid with ascorbic acid and evaluation of its in vitro activity on MC3T3-E1", THESIS GRADUATE SCHOOL, YONSEI UNIVERSITY, 1 January 2009 (2009-01-01), pages 1 - 58, XP055826638
- CHOI HO-IL ET AL: "A novel L-ascorbic acid and peptide conjugate with increased stability and collagen biosynthesis", vol. 42, no. 11, 30 November 2009 (2009-11-30), KR, pages 743 - 746, XP055951217, ISSN: 1976-6696, Retrieved from the Internet <URL:http://koreascience.or.kr/article/JAKO200910103425982.pdf> DOI: 10.5483/BMBRep.2009.42.11.743
- BAYER ILKER S.: "Hyaluronic Acid and Controlled Release: A Review", MOLECULES, vol. 25, no. 11, 1 June 2020 (2020-06-01), DE, pages 2649, XP055951193, ISSN: 1433-1373, DOI: 10.3390/molecules25112649
- TRIPATHI RAMA ET AL: "L-Ascorbic Acid in Organic Synthesis: An Overview", CURRENT ORGANIC CHEMISTRY, vol. 13, no. 1, 1 January 2009 (2009-01-01), NL, pages 99 - 122, XP055951191, ISSN: 1385-2728, DOI: 10.2174/138527209787193792
- GARRE AURORA ET AL: "Antiaging effects of a novel facial serum containing L-ascorbic acid, proteoglycans, and proteoglycan-stimulating tripeptide: ex vivo skin explant studies and in vivo clinical studies in women", CLINICAL. COSMETIC AND INVESTIGATIONAL DERMATOLOGY, vol. Volume 11, 1 May 2018 (2018-05-01), United Kingdom, pages 253 - 263, XP055951215, ISSN: 1178-7015, DOI: 10.2147/CCID.S161352
- WACH RADOSLAW A ET AL: "Hydroxyl radical-induced crosslinking and radiation-initiated hydrogel formation in dilute aqueous solutions of carboxymethylcellulose", CARBOHYDRATE POLYMERS, APPLIED SCIENCE PUBLISHERS , LTD BARKING, GB, vol. 112, 11 June 2014 (2014-06-11), pages 412 - 415, XP029045587, ISSN: 0144-8617, DOI: 10.1016/J.CARBPOL.2014.06.007
- WACH, R. A.: "Hydroxyl radical-induced crosslinking and radiation- initiated hydrogel formation in dilute aqueous solutions of carboxymethyl cellulose", CARBOHYDRATE POLYMERS, 2014, pages 412 - 415, XP029045587
- DONGJEONG PARK: "Conjugation of hyaluronic acid with ascorbic acid and evaluation of its in vitro activity on MC 3T3-E1", THESIS GRADUATE SCHOOL, YONSEI UNIVERSITY, 2009, pages 1 - 58, XP055826638
- YOUNG MIN SHIN, WOO-JIN KIM, YONG-SOO KIM, SUN-YOUNG JO, JONG-SEOK PARK,HUI-JEONG GWON, YOUN-MOOK LIM, YOUNG-CHANG NHO: "Modulation of Hyaluronic Acid Properties by Electron Beam Irradiation", JOURNAL OF RADIATION INDUSTRY, vol. 5, no. 2, 30 November 2010 (2010-11-30), Korea, pages 159 - 164, XP009528927

## Description

### TECHNICAL FIELD

The present disclosure relates to a method for preparing pure cross-linked hyaluronic acid nanoparticles, which have biocompatibility without a cross-linking agent, a conjugate of ascorbic acid and hyaluronic acid nanogels, a cosmetic composition comprising the conjugate, and a food composition comprising the conjugate.

### BACKGROUND ART

Hyaluronic acid is an ingredient that constitutes skin cells with elastin and collagen, is called a moisture storage capable of storing 1000 ml of moisture per 1 g, and has been widely used as a cosmetic material.

Nanoparticles such as pure hyaluronic acid hydration gels which are usable *in vivo* and biocompatible are required for being used as a contrast agent thereof and *in vivo.* A study of using hyaluronic acid to be cross-linked and converted into a nanoparticle form is still in an early stage and a study of synthesizing nanoparticles without using a cross-linking agent is hardly reported.

Meanwhile, there is provided a method for preparing nanoparticles by irradiating a hyaluronic acid aqueous solution with an electron beam (Korean Patent Registration No. 10-1893549). See, also, for example, US 2018/327554 A1 which describes generally forming of biocompatible nanoparticles by irradiation of an electron beam to an aqueous solution comprising at least one polysaccharide, a derivative thereof, and a polyethylene glycol.

However, since the hyaluronic acid concentration is high in conditions in which the nanoparticles are prepared, it is not economic, and only nanoparticles having a diameter of more than 100 nm are prepared, and thus, there is a limit to be used in various fields.

Also, the following publications describe generally formation of ascorbic acid conjugates: Park DongJeong "Conjugation of hyaluronic acid with ascorbic acid and evaluation of its in vitro activity on MC3T3-E1 ", Thesis Graduate School, Yonsei university, 1 January 2009 (2009-01-01), pages 1-58, XP055826638;

Choi Ho-Il et al: "A novel L-ascorbic acid and peptide conjugate with increased stability and collagen biosynthesis", BMB reports, vol. 42, no. 11, 30 November2009 (2009-11-30), pages 743-746, XP055951217, KR ISSN: 1976-6696, DOI: 10.5483/BMBRep.2009.42.11.743 Retrieved from the Internet: URL:http://koreascience.or.kr/article/JAK0200910103425982.pdf.

Generally, use of hyaluronic acid derivatives for treating various conditions and methods of making such derivatives has been reported in the following publications:
Bayer Ilker s.: "Hyaluronic acid and controlled release: a review", molecules, vol. 25, no. 11, 1 June 2020 (2020-06-01), page 2649, XP055951193, DE ISSN: 1433-1373, DOI: 10.3390/molecules25112649;
Tripathi Rama et al: "L-Ascorbic Acid in Organic Synthesis: An Overview", Current Organic Chemistry, vol. 13, no. 1, 1 January 2009 (2009-01-01), pages 99-122, XP055951191, NL ISSN: 1385-2728, DOI: 10.2174/138527209787193792;
Garre aurora et al: "Antiaging effects of a novel facial serum containing L-ascorbic acid, proteoglycans, and proteoglycan-stimulating tripeptide: ex vivo skin explant studies and in vivo clinical studies in women", clinical. cosmetic and investigational dermatology, vol. Volume 11, 1 May 2018 (2018-05-01), pages 253-263, XP055951215, United Kingdom ISSN: 1178-7015, DOI: 10.2147/CCID.S161352; and
Wach Radoslaw a et al: "Hydroxyl radical-induced crosslinking and radiation-initiated hydrogel formation in dilute aqueous solutions of carboxymethylcellulose", Carbohydrate polymers, Applied science publishers, LTD Barking, GB, vol. 112, 11 June 2014 (2014-06-11), pages 412-415, XP029045587, ISSN: 0144-8617, DOI: 10.1016/J.CARBPOL.2014.06.007.

See also US 2012/283428 A1 describing a hyaluronic acid derivative for treating atopic dermatitis and a method for manufacturing the hyaluronic acid derivative.

### DISCLOSURE

### TECHNICAL PROBLEM

Accordingly, the present disclosure provides a new method for preparing biocompatible and pure hyaluronic acid nanoparticles or nanogels by recognizing the conventional problems and needs.

The present disclosure provides a method capable of forming cross-linked hyaluronic acid nanoparticles on an aqueous solution other than an organic solvent without a separate cross-linking agent and provides a method capable of controlling sizes of nanoparticles.

### TECHNICAL SOLUTION

The invention is defined in the appended claims. The present disclosure provides a method for preparing cross-linked hyaluronic acid nanoparticles, comprising forming intermolecular or intramolecular cross-linking of hyaluronic acid by irradiating a hyaluronic acid aqueous solution with an electron beam without a separate cross-linking agent, and adjusting the pH of the hyaluronic acid aqueous solution, thereby controlling the size of the hyaluronic acid nanoparticles, wherein the pH of the hyaluronic acid aqueous solution is 1 to 4.

In the present disclosure, the nanoparticles refer to particles having sizes of several to hundreds of nanometers (nm, material with one billionth of a meter). In the present disclosure, the nanoparticles are characterized to have particle sizes of 1 to 100 nm, but are not limited thereto. In the present disclosure, the nanoparticle is a concept including a nanogel or nanohydrogel, and hereinafter, the nanoparticle, and the nanogel or nanohydrogel are all used to mean the nanoparticle according to the present disclosure.

The hyaluronic acid aqueous solution is acidic, and may further include HClO₄ in the hyaluronic acid-containing solution for an acidic aqueous solution. As described in detail and proved below, when the hyaluronic acid aqueous solution is acidic or the acid is added to the hyaluronic acid aqueous solution, it is newly provided that as compared with if not, high nanoparticle synthesis efficiency is increased. It is also provided that the sizes of the nanoparticles may be unexpectedly controlled due to adjustment of pH and adjustment of an added concentration of an acidic compound.

The hyaluronic acid is widely distributed in the cell-matrix in the connective tissue to form an adhesive and elastic solution under a physiological condition and is a main component of the extracellular matrix which is known to physically protect tissues such as iris and retina, vascular endothelial cells and epithelial cells, etc. and provide a buffer role. The hyaluronic acid is not limited to physical properties, shapes, size, and the like, and is preferably sterilized. In addition, the hyaluronic acid may have an average molecular weight of 5 to 5000 kDa, preferably an average molecular weight of 5 to 3000 kDa. In addition, the hyaluronic acid may be used in the form of a pharmaceutically acceptable salt, wherein the pharmaceutically acceptable salt includes a sodium salt, a potassium salt, a calcium salt, etc., and preferably a sodium salt. Further, derivatives of hyaluronic acid may also be used, and these derivatives may be specifically hyaluronic acids chemically modified by cross-linking, sulfuration, esterification or amino acid binding.

In the present disclosure, the pH of the hyaluronic acid aqueous solution is 1 to 4, and preferably pH 1.5 to 3.5.

In the preparation method of the hyaluronic acid nanoparticles, the concentration of the hyaluronic acid aqueous solution may be 0.05 to 7% (w/v), preferably 0.1 to 5% (w/v).

The aqueous solution does not include a cross-linking agent and an organic solvent.

After irradiating the electron beam, dialysis and freeze-drying steps are further included.

The sizes of the hyaluronic acid nanoparticles are controlled by changing an irradiation dose of the electron beam and the sizes of the hyaluronic acid nanoparticles are reduced by increasing the irradiation dose of the electron beam.

In the preparation method of the hyaluronic acid nanoparticles, the electron beam may be irradiated at the irradiation dose of 0.5 to 300 kGy, preferably 1 to 250 kGy, most preferably 1 to 200 kGy.

In the present disclosure, a total irradiation energy intensity of the electron beam may be 0.1 MeV to 5 MeV. The total irradiation energy intensity of the electron beam may be preferably 0.5 MeV to 3 MeV, and most preferably 1.0 MeV to 2.5 MeV.

The sizes of the hyaluronic acid nanoparticles are controlled by adjusting pH of the aqueous solution and the sizes of the hyaluronic acid nanoparticles are increased by decreasing pH of the aqueous solution.

The preparation method of the hyaluronic acid may be a preparation method of hyaluronic acid nanoparticles having a diameter of 100 nm or less, preferably hyaluronic acid nanoparticles having a diameter of 50 nm or less, most preferably hyaluronic acid nanoparticles having a diameter of 30 nm or less.

The present disclosure provides intermolecular or intramolecular cross-linked nanoparticles of hyaluronic acid prepared by the inventive method.

The nanoparticles are characterized as hydrogels with a swelling property.

The nanoparticles are characterized by having tumor selectivity. In particular, the nanoparticles are characterized to have colon cancer or melanoma selectivity.

The nanoparticles are characterized to have a tumor-to-muscle ratio of higher than 8 times and a tumor-to-blood ratio of 1.5 times for the colon cancer tumor. The nanoparticles are characterized to have the tumor-to-muscle ratio of higher than 14 times and the tumor-to-blood ratio of higher than 3 times for the melanoma.

As another aspect of the present disclosure which is not included in the present disclosure as claimed a contrast agent is provided including the hyaluronic acid nanoparticles which are labeled with at least one labeling material selected from the group consisting of radioactive isotopes, organic fluorescent materials, quantum dots as inorganic materials, a magnetic resonance image contrast agent, a computed tomography contrast agent, a positron tomography contrast agent, an ultrasonic contrast agent, a fluorescent contrast agent, and a shape shift material.

A ligand compound conjugated to the nanoparticles and a radioactive isotope coordinately bonded to the ligand compound are included.

The ligand compound is characterized as at least one of NODA-GA-NH₂, DOTA-GA, DOTA, TETA, and NOTA.

The radioactive isotope is characterized as at least one of ¹¹C, ¹³N, ¹⁵O, ¹⁸F, ³⁸K, ⁶²Cu, ⁶⁴Cu, ⁶⁸Ga, ⁸²Rb, ¹²⁴I, ⁸⁹Zr, ^{99m}Tc, ¹²³I, ¹¹¹In, ⁶⁷Ga, ¹⁷⁷Lu, ²⁰¹Tl, ^{117m}Sn, ¹²⁵I, ¹³¹I, ¹⁶⁶Ho, ¹⁸⁸Re, ⁶⁷Cu, ⁸⁹Sr, ⁹⁰Y, ²²⁵Ac, ²¹³Bi, and ²¹¹At.

As another aspect, the present disclosure provides a conjugate of ascorbic acid and hyaluronic acid nanogels wherein the hyaluronic acid nanogels are prepared according to the inventive method.

In the present disclosure, the "conjugate" refers to a compound having two or more compounds linked to each other. The linkage may be achieved by a non-covalent or covalent bond. That is, the conjugate of the present disclosure means that ascorbic acid and hyaluronic acid are directly or indirectly linked to each other by a non-covalent bond or covalent bond. Preferably, the conjugate of the present disclosure may mean that a hydroxyl group of ascorbic acid forms a bond to a carboxylic group of hyaluronic acid through an alkyl group of C1 to C5 to be firmly linked by a covalent bond.

In the present disclosure, the "ascorbic acid" is a material having an antioxidant effect represented by the following Chemical Formula 1 and called Vitamin C as a water-soluble vitamin and has been used in various industrial applications including pharmaceutical preparations, feed, inhibition of accumulation of melanin pigments, medical drugs, and cosmetics. However, the ascorbic acid as a very unstable compound is unstable under an oxidation condition and a thermal condition to lose the activity when exposed to heat, air, and light in the preparation process, and the activity thereof is lost in a distribution process, and thus, there is a large limit on utilization.

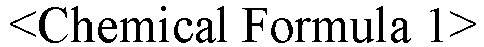

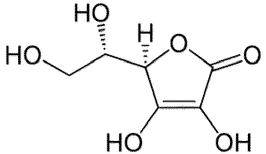

In the present disclosure, unless otherwise stated, the term "hyaluronic acid nanogel" refers to hyaluronic acid having various chain lengths and charge states as well as various chemical modifications including cross-linking, all variants of hyaluronate or hyaluronan, and hydrogels having a nano-meter size by cross-linking the variants. That is, the hyaluronic acid nanogel collectively includes various hyaluronate-derived nanogels of hyaluronic acid having various opposite ions, such as sodium hyaluronate. For example, various derivative-derived nanogels of hyaluronic acid are collectively included by the term, such as oxidation, such as oxidation to -CHO and/or -COOH of a -CH₂OH group; periodate oxidation of adjacent hydroxyl groups selectively involving reduction, such as reduction of forming imine by reduction to -CH₂OH of -CHO and involving reduction to amine; sulphation; selectively deamidation or deamidation involving formation of amide together with a new acid; esterification; cross-linking; substitution with various compounds, using binding by assistance of a cross-linking agent or carbodiimide, including binding of hyaluronic acid with various molecules such as proteins, peptides, and active medical ingredients; and deacetylation.

In the present disclosure, the preparation method of the hyaluronic acid nanogel is not particularly limited so long as the nanogel is a hydrogel of a nano-meter size formed by a hyaluronic acid molecule as a basic unit. In the art, the method for preparing the hyaluronic acid hydrogel having the nano-meter size is well known, and for example, a preparation method using a cross-linking agent, a preparation method using an electron beam, etc. may be referred to, but most preferably, the hyaluronic acid nanogel may be prepared by irradiating an electron beam to the aforementioned hyaluronic acid aqueous solution.

In the present disclosure, the conjugate of the ascorbic acid and the hyaluronic acid nanogels may be represented by the following Chemical Formula 2.

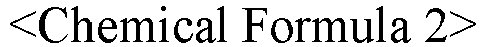

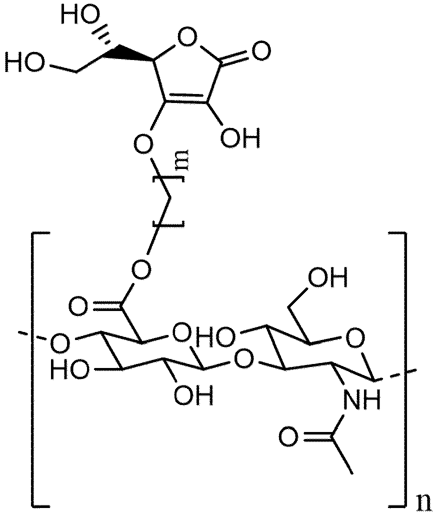

Wherein, m is 1 to 5.

In the conjugate provided by the present disclosure, a ratio of an ascorbic acid molecule to a carboxyl acid group of the hyaluronic acid molecule may be 1 to 5:1, preferably 1 to 4:1, much more preferably 2 to 4:1, and most preferably 2 to 3:1.

The conjugate of the ascorbic acid and the hyaluronic acid nanogels may be sequentially prepared according to the following Reaction Formulas 1 to 3.

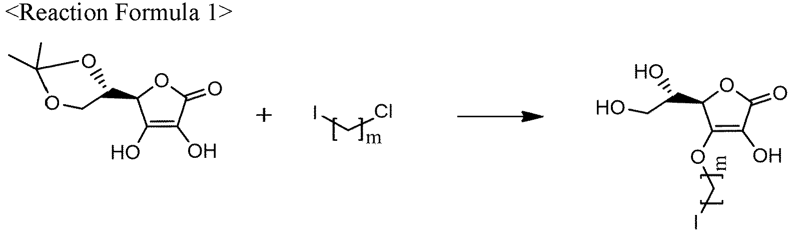

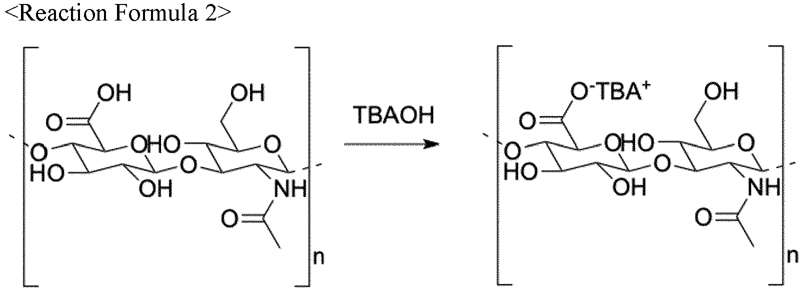

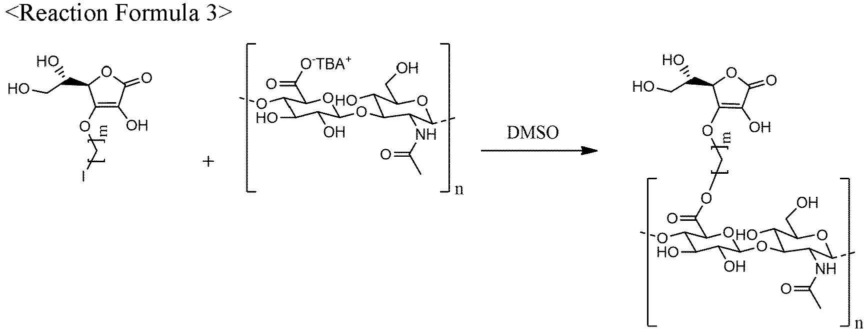

Wherein, m is 1 to 5.

In Reaction Formulas 2 and 3 of the present disclosure, the hyaluronic acid may be induced by cross-linking after Reaction Formulas 2 and 3 to form nanogels, and after the hyaluronic acid is prepared in a nanogel form according to a known method or a preparation method of hyaluronic acid nanogels provided by the present disclosure, Reaction Formulas 2 and 3 may also be applied.

Preferably, in Reaction Formulas 2 and 3 of the present disclosure, after the hyaluronic acid is prepared in a nanogel form according to a known method or a preparation method of hyaluronic acid nanogels provided by the present disclosure, Reaction Formulas 2 and 3 may be applied.

According to an embodiment of the present disclosure, it was confirmed that the ascorbic acid included in the conjugate has significantly improved stability and then the physiological activity of ascorbic acid is equally exhibited even after the ascorbic acid is left under a very harsh condition. Accordingly, the conjugate provided by the present disclosure is characterized in that the storage stability of ascorbic acid is improved.

In another aspect, the present disclosure provides a cosmetic composition for antioxidation, skin whitening, wrinkle improvement or skin anti-aging comprising the ascorbic acid and the hyaluronic acid nanogels as active ingredients.

Further, the present disclosure provides a cosmetic composition for antioxidation, skin whitening, wrinkle improvement or skin anti-aging (essentially) consisting of the ascorbic acid and the hyaluronic acid nanogels as active ingredients.

The ingredients included in the cosmetic composition of the present disclosure include ingredients which are generally used in the cosmetic composition as active ingredients in addition to the active ingredients, and for example, include general adjuvants such as antioxidants, stabilizers, solubilizers, vitamins, pigments, and fragrances, and carriers.

The cosmetic composition of the present disclosure may be prepared even in any formulation commonly prepared in the art, and may be formulated by, for example, a solution, a suspension, an emulsion, a paste, a gel, a cream, a lotion, a powder, a soap, a surfactant-containing cleansing, oil, a powder foundation, an emulsion foundation, a wax foundation, a spray, and the like, but is not limited thereto. More specifically, the cosmetic composition of the present disclosure may be prepared by softener (skin), nourishing toner (milk lotion), nourishing cream, massage cream, essence, eye cream, cleansing cream, cleansing foam, cleansing water, pack, spray, or powder formulations.

Preferably, the cosmetic composition of the present disclosure may be W/O cream, O/W cream, O/W essence and hydrogel formulations, and most preferably a hydrogel formulation, but is not limited thereto.

When the formulation of the present disclosure is the paste, cream, or gel, as a carrier ingredient, animal oils, vegetable oils, wax, paraffin, starch, tragacanth, cellulose derivatives, polyethylene glycol, silicone, bentonite, silica, talc, zinc oxide or the like may be used.

When the formulation of the present disclosure is the powder or spray, as the carrier ingredient, lactose, talc, silica, aluminum hydroxide, calcium silicate or polyamide powder may be used, and particularly, in the case of the spray, additionally, a propellant such as chlorofluorohydrocarbon, propane/butane or dimethyl ether may be included.

When the formulation of the present disclosure is the solution or emulsion, as the carrier ingredient, a solvent, a solubilizing agent or an emulsifying agent may be used. For example, the carrier ingredient includes water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or fatty acid ester of sorbitan.

When the formulation of the present disclosure is the suspension, as the carrier ingredient, a liquid diluent such as water, ethanol or propylene glycol, a suspension such as ethoxylated isostearyl alcohol, polyoxyethylene sorbitol ester and polyoxyethylene sorbitan ester, microcrystalline cellulose, aluminum metahydroxide, bentonite, agar, tragacanth, or the like may be used.

When the formulation of the present disclosure is the surfactant-containing cleansing, as the carrier ingredient, aliphatic alcohol sulfate, aliphatic alcohol ether sulfate, sulfosuccinic acid monoester, isethionate, imidazolinium derivatives, methyltaurate, sarcosinate, fatty acid amide ether sulfate, alkylamido betaine, aliphatic alcohol, fatty acid glyceride, fatty acid diethanolamide, vegetable oil, lanolin derivatives, ethoxylated glycerol fatty acid ester, or the like may be used.

In the present disclosure, the composition may have an antioxidant activity. The antioxidation means an effect of suppressing oxidation, and in the human body, a prooxidant and an antioxidant are balanced, but when this balanced state is imbalanced by various factors and biased toward the promotion of oxidation, oxidative stress is induced to cause potential cell damage and pathological diseases.

In one embodiment of the present disclosure, it was confirmed that the composition exhibited an excellent DPPH free radical scavenging activity.

The term "whitening" used herein means whitening the skin, and means all actions of inhibiting or preventing the skin pigmentation of melanin by inhibiting the synthesis of melanin. In the present disclosure, the composition may inhibit the activity of tyrosinase. The tyrosinase is a metal-containing protein containing a pair of copper ions in an active site and refers to an enzyme involved in a process in which in a production path of melanin, L-tyrosine is converted to 3,4-dihydroxyphenyl alanine (L-DOPA) through a hydroxylated reaction, oxidized to phenylalanine-3,4-quinen (dapaquinone) again, and then synthesized to melanin through various intermediates such as dopachrome, indole-5,6-quinen, etc.

In one embodiment of the present disclosure, it was confirmed that the composition had a tyrosinase inhibitory activity in a concentration-dependent manner and thus, it can be seen that the composition had a whitening activity.

In the present disclosure, the composition may be characterized by inhibiting the production of melanin. The melanin is made by a dye of black brown granules and is made by a cell organelle called melanosomes in melanocytes. The melanin is present in skin, hair, eye, and the like, and a skin color is determined according to the amount of melanin, and the more the amount of melanin, the darker the skin color.

In one embodiment of the present disclosure, it was confirmed that the composition had a melanin production inhibitory effect in a concentration-dependent manner and thus, it can be seen that the composition had a whitening activity.

The term "wrinkles" used herein refers to fine lines occurring by declining in the skin, and may be caused by genetic factors, reduction in collagen existing in the skin dermis, external environments, etc. In the present disclosure, the composition may be characterized by promoting collagen biosynthesis. The collagen is present in a collective tissue such as bone, cartilage, skin, and tendon. When the skin is continuously exposed to external stimuli, such as ultraviolet rays, collagen fibers involved in skin elasticity are damaged, resulting in the aging of the skin.

In one embodiment of the present disclosure, it was found that the composition may exhibit a wrinkle improvement effect by increasing the collagen biosynthesis in a concentration-dependent manner.

The term "skin aging" used herein shows symptoms such as reduction in elasticity of the skin, reduction in shine, generation of wrinkles, decreased regeneration, or severe drying, and may be caused by the passage of time, external environments, etc.

In one embodiment of the present disclosure, it was found that the composition may promote the proliferation of fibroblasts to improve regeneration and elasticity of damaged skin.

In another aspect, the present disclosure provides a food composition for antioxidation, skin whitening, wrinkle improvement or skin anti-aging comprising the conjugate of the ascorbic acid and the hyaluronic acid nanogels as active ingredients.

Further, the present disclosure provides a food composition for antioxidation, skin whitening, wrinkle improvement or skin anti-aging (essentially) consisting of the conjugate of the ascorbic acid and the hyaluronic acid nanogels as active ingredients.

The food composition according to the present disclosure includes all forms such as functional foods, nutritional supplements, health foods, and food additives. The types thereof may be prepared in various forms according to a general method known in the art.

For example, as the health food, the food composition itself of the present disclosure may be prepared in the form of tea, juice, and drinks, to be drunk, or taken in by granulation, encapsulation and powder. Further, the food composition of the present disclosure may be prepared in the form of a composition mixed with a known material or active ingredient known as an effect of antioxidation, skin whitening, wrinkle improvement or skin anti-aging.

Further, the functional food may be prepared by adding the food composition of the present disclosure to beverages (including alcoholic beverages), fruits and processed foods thereof (e.g., canned fruit, bottled food, jam, marmalade, etc.), fish, meat and processed foods thereof (e.g., ham, sausage, corned beef, etc.), bread and noodles (e.g., udon, buckwheat noodles, ramen, spaghetti, macaroni, etc.), fruit juice, various drinks, cookies, sweets, dairy products (e.g., butter, cheese, etc.), edible vegetable oil, margarine, vegetable protein, retort food, frozen food, various seasonings (e.g., soybean paste, soy sauce, sauce, etc.), etc.

The preferred content of the food composition according to the present disclosure is not limited thereto, but is preferably 0.01 to 50 wt% of the total weight of the finally prepared food. In order to use the food composition of the present disclosure in the form of food additives, the food composition may be used in the form of powder or concentrate.

Further, disclosed but not included in the claims is a use of a conjugate of the ascorbic acid and the hyaluronic acid nanogels for preparing a preparation for antioxidation, skin whitening, wrinkle improvement, or skin anti-aging.

Further, disclosed but not included in the claims is a method for antioxidation, skin whitening, wrinkle improvement, or skin anti-aging comprising administering an effective dose of the composition comprising the conjugate of the ascorbic acid and the hyaluronic acid nanogels as active ingredients, to a subject in need thereof

The term 'effective dose' means an amount which exhibits effects of improving, treating, preventing, detecting, and diagnosing of diseases associated with antioxidation, skin whitening, wrinkle improvement, or skin anti-aging, or inhibiting or alleviating diseases associated with antioxidation, skin whitening, wrinkle improvement, or skin anti-aging when administered to the subject. The 'subject' may be animals, preferably, mammals, particularly animals including humans and may also be cells, tissues, and organs derived from animals. The subject may be a patient requiring the effects.

The term 'treatment' comprehensively refers to improving diseases associated with antioxidation, skin whitening, wrinkle improvement, or skin anti-aging or symptoms of the diseases associated with antioxidation, skin whitening, wrinkle improvement, or skin anti-aging. The treatment may include treating or substantially preventing these diseases, or improving the conditions thereof and include alleviating, treating or preventing a symptom or most of symptoms derived from the diseases, but is not limited thereto.

The term 'comprising' is used in the same manner as 'containing' or 'characterizing,' and does not exclude additional ingredients or steps of the method which are not mentioned in the composition or the method. The term 'consisting of' means excluding additional elements, steps or ingredients, etc., unless otherwise noted. The term 'essentially consisting of' or 'essentially comprising' means including ingredients or steps that do not substantially affect basic properties thereof in addition to the described ingredients or steps within the range of the composition or the method.

### ADVANTAGEOUS EFFECTS

According to the present disclosure, there is provided a method for preparing pure hyaluronic acid nanoparticles on an aqueous solution without a cross-linking agent and an organic solvent.

### DESCRIPTION OF DRAWINGS

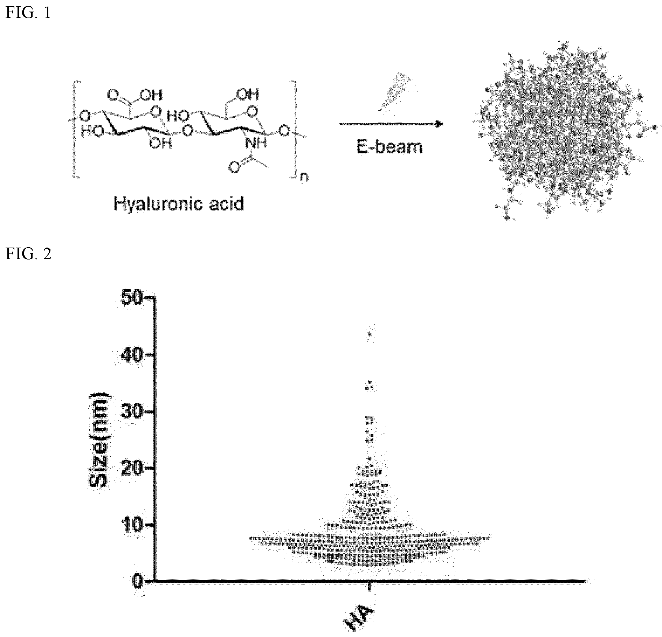

FIG. 1 is a schematic diagram illustrating that hyaluronic acid polymers are cross-linked by an electron beam to form hyaluronic acid particles.
FIGS. 2 and 3 are graphs showing a size distribution of hyaluronic acid in Example 1 of the present dislcosure.
FIG. 4 illustrates a bar graph of particle sizes of nanogels according to an irradiation dose of an electron beam when the electron beam is irradiated to 100 kDa of a hyaluronic acid (HA3) 0.5% (W/V) aqueous solution (pH 2.0 to 2.4).
FIG. 5 is a result of DLS measurement of one hyaluronic acid nanoparticle in a sample of Example 1 of the present disclosure.
FIG. 6 is a photograph of samples obtained in a powder form after synthesizing hyaluronic acid nanoparticles in large quantities and freeze-drying the nanoparticles.
FIG. 7 is a TEM image of hyaluronic acid nanoparticles prepared according to Example 1 of the present disclosure.
FIG. 8 is a photograph showing an experimental result for hydrogel properties of hyaluronic acid nanoparticles prepared according to Example 1 of the present disclosure.
FIG. 9 is a schematic diagram illustrating a process of preparing a chelate compound by conjugating a bifunctional chelate NODA-GA-NH₂ in hyaluronic acid nanoparticles of Example 2 of the present disclosure.
FIG. 10 is a schematic diagram illustrating a process of radiolabeling the hyaluronic acid nanoparticles of Example 2 of the present disclosure with Cu-64.
FIG. 11 is a diagram illustrating a radiochemical purity result of hyaluronic acid nanoparticles radiolabeled with Cu-64 of Example 2 of the present disclosure.
FIG. 12 is a time-dependant radio-TLC result of analyzing stability in a phosphate buffer saline (PBS) and fetal bovine serum (FBS) of hyaluronic acid nanoparticles radiolabeled with Cu-64 of Example 2 of the present disclosure.
FIG. 13 illustrates a result of an *in vivo* distribution verifying experiment in a normal mouse of hyaluronic acid nanoparticles radiolabeled with Cu-64 of Example 2 of the present disclosure.
FIG. 14 is a result of an *in vivo* distribution verifying experiment even for hyaluronic acid nanoparticles obtained by precipitating with an organic solvent other than a freeze-drying process of generally obtaining synthesized hyaluronic acid nanoparticles as a final sample.
FIG. 15 is a result of an *in vivo* distribution verifying experiment in a tumor model using colon tumor cells (CT26) in the hyaluronic acid nanoparticles of Example 2 of the present disclosure.
FIG. 16 is a result of an *in vivo* distribution verifying experiment in a tumor model using skin cancer cells (melanoma/B16F10) in the hyaluronic acid nanoparticles of Example 2 of the present disclosure.
FIG. 17 is a nuclear medicine image for a tumor model using hyaluronic acid nanoparticles labeled with radioactive isotopes Cu-64.
FIG. 18 is a diagram illustrating a process of synthesizing an ascorbic acid derivative by binding 1-chloro-3-iodopropane in an OH group at position 3 of ascorbic acid protected in an acetal form, substituting a chloro group (Cl) with an iodo group (I), and removing an acetal group as a protective group.
FIG. 19 is a diagram illustrating a process of substituting a carboxylic group of a hyaluronic acid nanogel with a tetrabutyl ammonium (TBA) group.
FIG. 20 is a diagram illustrating a process of synthesizing a hyaluronic acid nanogel derivative bound with ascorbic acid in a powder form by stirring an ascorbic acid derivative substituted with an iodo group with hyaluronic acid nanogels obtained in a TBA form under a DMSO solvent for about 12 hours and adding acetone to a reaction solution.
FIGS. 21A and 21B are diagrams of confirming whether a hyaluronic acid nanogel derivative bound with ascorbic acid is prepared by NMR (FIG. 21A) and HPLC chromatogram (FIG. 21B).
FIG. 22 illustrates a result of evaluating stability by analyzing a hyaluronic acid nanogel derivative (HA-VitC bound with ascorbic acid and an ascorbic acid (AA) aqueous solution using HPLC after left at 50°C for 48 hours, respectively.
FIGS. 23A and 23B illustrate results of evaluating the ability of inhibiting melanin production and the ability of inhibiting tyrosinase activity of each experimental substance.
FIGS. 24A and 24B are diagrams illustrating a result of evaluating the free radical scavenging ability of each experimental substance by DPPH assay (FIG. 24A) and a result of quantifying the result by a graph (FIG. 24B).
FIGS. 25A and 25B are results of evaluating the tyrosinase inhibitory ability of each experimental substance.
FIG. 26 illustrates a result of evaluating the ability of promoting fibroblast proliferation of each experimental substance.
FIG. 27 illustrates a result of evaluating an effect of each experimental substance on collagen biosynthesis.

### MODES FOR THE INVENTION

Various aspects of the present invention are defined in the independent claims. Preferred embodiments of the present invention are given in the dependent claims. In describing each drawing, like reference numerals were used for like components.

Terms used in the present application are used only to describe specific embodiments, and are not intended to limit the present disclosure. A singular form may include a plural form unless otherwise clearly indicated in the context. In the present application, it should be understood that term "including" or "having" indicates that a feature, a number, a step, an operation, a component, a part or a combination thereof described in the specification is present, but does not exclude a possibility of presence or addition of one or more other features, numbers, steps, operations, components, parts or combinations thereof, in advance.

Unless otherwise contrarily defined, all terms used herein including technological or scientific terms have the same meanings as those generally understood by a person with ordinary skill in the art. Terms which are defined in a generally used dictionary should be interpreted to have the same meaning as the meaning in the context of the related art, and are not interpreted as an ideal meaning or excessively formal meanings unless clearly defined in the present application.

### Example 1: Nanoparticle synthesis analysis according to molecular weight size, electron beam condition and acid concentration of hyaluronic acid

In the case of an electron-beam irradiation experiment using hyaluronic acid, three types of hyaluronic acids having different molecular weights below were used and the experiment was performed in an acidic aqueous solution condition.

**Table 1**

| | |
|---|---|
| HA1 | Oligo Hyaluronic Acid (8 kDa) |
| HA2 | High molecular weight sodium hyaluronate (2.5 MDa) |
| HA3 | Low molecular weight sodium hyaluronate (100 kDa) |

For reference, through the prior study (Korean Patent Registration No. 1893549), the present inventors confirmed that nanogels of 380 nm were generated when an electron beam of 50 kGy was irradiated to 10 kDa of a hyaluronic acid 1%(w/v) aqueous solution, and nanogels of 108 nm were generated when an electron beam of 200 kGy was irradiated to a hyaluronic acid 5%(w/v) aqueous solution. However, in other conditions, the nanogels were not formed, and even in the case of the nanoparticles prepared under the conditions, it was found that there was a limit to the industrial utilization because the size was relatively large.

Accordingly, the present inventors attempted a method of irradiating an electron beam to a hyaluronic acid aqueous solution under an acidic condition to provide nanogels having very small particle sizes as well as capable of being prepared with economical costs under a lower concentration condition of the hyaluronic acid aqueous solution while using hyaluronic acid (HA1) of a molecular weight (8 kDa) similar to the prior study.

Specifically, HClO₄ was added to the HA1 aqueous solution of a concentration (w/v%) shown in Table 2 below to adjust pH to 1.6 to 2.1, and then the electron beam was irradiated at energy of 1 MeV with an irradiation dose (kGy) shown in Table 2 below.

The result thereof was shown in Table 2 below.

As shown in Table 2 below, in various electron beam irradiation energy, when hyaluronic acid (HA1) at various concentrations was used and additional acid was added, conditions capable of synthesizing small nanoparticles having a size of 3 to 4 nm with a low polydispersity index were confirmed.

**Table 2**

| | |
|---|---|
| HA1 5%, pH 2.4, 1Mev, 200kGy (3.9nm, Pdl 0.21) | - Size 3-4nm |
| HiA1 1%, pH 2.0, 1Mev, 150kGy (3.1nm, Pdl 0.21) | - Polydispersity index (Pdl) < 0.26 |
| HA1 1%, pH 2.4, 1Mev, 150kGy (2.9nm, Pdl 0.18) | |
| HA1 1%, pH 2.4, 1Mev, 200kGy (4nm, Pdl 0.22) | - Reproducibility |
| HA1 0.5%, pH 2.0, 1Mev, 200kGy (2.9nm, Pdl 0.22) | |
| HA1 0.2%, pH 2.0, 1Mev, 150kGy (4.6nm, Pdl 0.25) | |
| HA1 0.2%, pH 3.2, 1Mev, 200kGy (2.9nm, Pdl 0.19) | |

Next, in the case of HA2, it was confirmed that when acid A (HClO₄) was added to lower pH to 1.8 to 2.2 and the electron beam was irradiated, the nanoparticles were generated well, and it was confirmed that as the concentration of the added acid was increased, the size of the particles tended to be increased.

**Table 3**

| | 1MeV |
|---|---|
| HA2 0.1%, pH 2.2 | |
| HA2 0.1%, pH 2.0 | 3kGy: 10nm; |
| | 50kGy:5.6nm; |
| | 150kGy:8.5nm |
| HA2 0.1%, pH 1.8 | 1kGy: 10.3nm; |
| | 50kGy: 6.9nm; |
| | 150kGy:9.7nm; |
| | 200kGy:7.8nm |
| | |
| HA2 0.2%, pH 2.2 | 50kGy:6.4nm; |
| | 150kGy:4.8nm |
| HA2 0.2%, pH 2.0 | 3kGy: 11.3nm; |
| | 50kGy: 8nm; |
| | 100kGy: 7nm; |
| | 150kGy:7.4nm; |
| | 200kGy: 6.7 nm |
| HA2 0.2%, pH 1.8 | 1kGy: 14nm; |
| | 50kGy: 10.6nm; |
| | 100kGy: 6.2nm; |
| | 200kGy: 8.4nm |

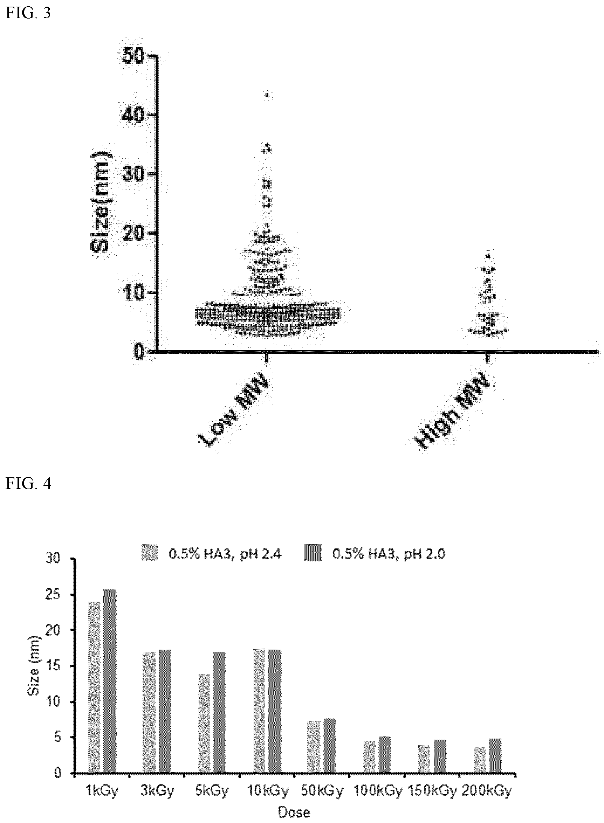

Next, even in an experiment using HA3, it was confirmed that nanoparticles were generated when an acid was added. The result thereof was illustrated in FIG. 4. Further, it was confirmed that as the concentration of the added acid was generally increased, the size of the particles tended to be increased, and it was confirmed that as the irradiation dose of electron beam was increased, the size of the particles tended to be decreased.

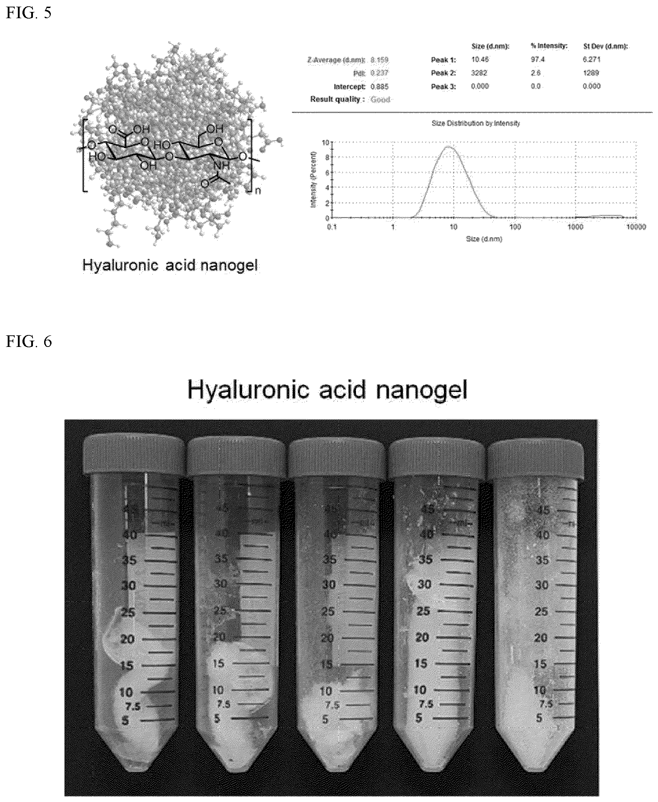

As shown in FIG. 5, it was confirmed that one particle of the sample synthesized in Example 1 had a size of 8.1 nm through DLS.

The hyaluronic acid nanoparticles showing the result were synthesized in large quantities, and a sample was obtained in a powder form after a freeze-drying process, as shown a photograph of FIG. 6.

In the following experiment, nanoparticles were prepared and used by irradiating 100 kGy of an electron beam (irradiation energy of 2.5 MeV) in a 1% (w/v) HA3 aqueous solution at pH 2.0.

### (1) Structural analysis of nanoparticles

The study of the synthesized hyaluronic acid nanoparticles was conducted not only to measure the size of the nanoparticles through DLS, but also to directly confirm a morphological shape of the actually synthesized nanoparticles through a transmission electron microscope (TEM).

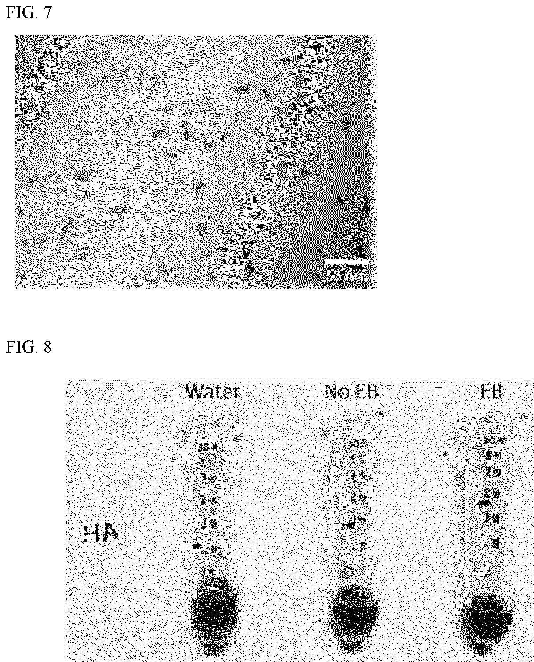

The nanoparticles were dropped to 2 to 3 drops on a copper grid coated with carbon, subjected to a dying process using uranyl acetate, and then analyzed using TEM illustrated in FIG. 7 after fully drying moisture with a sufficient time.

Like the results of DLS, it was confirmed that the nanoparticles had small sizes of approximately 10 nm or less, and it was confirmed that the shape of the particle was not clear, but kept an approximately round shape.

### (2) Characteristic Analysis of Hydrogel

An experiment of confirming whether the synthesize nanoparticle had a substantially swelling characteristic of a hydrogel was conducted, and the experiment was conducted using pure water and hyaluronic acid without irradiating an electron beam as a control.

The same amounts of hyaluronic acid nanoparticles and control were dissolved in 500 µL of water, respectively, and then centrifuged using a centrifugal filter with a separation membrane to check an amount of water dropped on each tube bottom, and in order to see a difference thereof well, the water dropped below the tube was dyed with green ink and then captured. It was illustrated in FIG. 8.

When confirmed the results, it can be confirmed that the amount of water dropped to the bottom of the tube after a centrifuging process is smaller in the nanoparticle sample obtained by irradiating the electron beam and a more solution is left in a supernatant portion. Through such a result, it can be confirmed that hyaluronic acid nanoparticles synthesized through the electron beam irradiation are further swelled than the control and have a characteristic as a hydrogel.

### Example 2: Application of hyaluronic acid nanogels

### (1) Evaluation of stability of hyaluronic acid nanogels conjugated with contrast agent

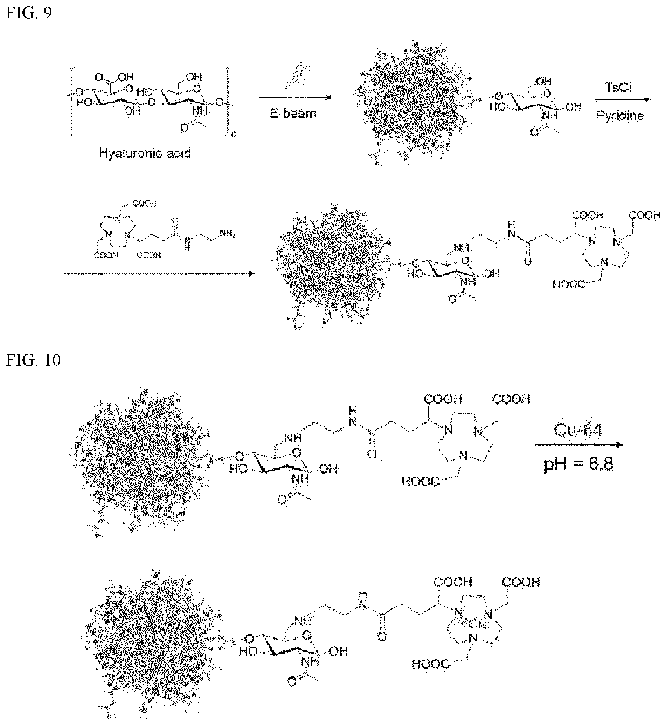

As shown in a schematic diagram of FIG. 9, an experiment of conjugating hyaluronic acid nanogels was conducted by using NODA-GA-NH₂, and the hyaluronic acid nanogels were dissolved in pyridine and then tosyl chloride was slowly dropped, the reaction was performed overnight. Thereafter, after NODA-GA-NH₂ was added, hot heat was applied and the reaction was performed for about 1 day, and then the hyaluronic acid nanogels conjugated with NODA-GA-NH₂ were separated by a centrifugal filter. Through the freeze-drying process, the hyaluronic acid nanogels conjugated with NODA-GA were obtained in a pure white powder form.

As shown in a schematic diagram of FIG. 10, radiolabeling using Cu-64 was performed, NODA-GA-hyaluronic acid nanogels were added in a pH 6.8 buffer to react with ⁶⁴CuCl₂ at appropriate temperature and time, and then the hyaluronic acid nanogels labeled with Cu-64 were purified through a centrifugal filter, and thereafter, radiochemical purity was confirmed by using Radio-TLC.

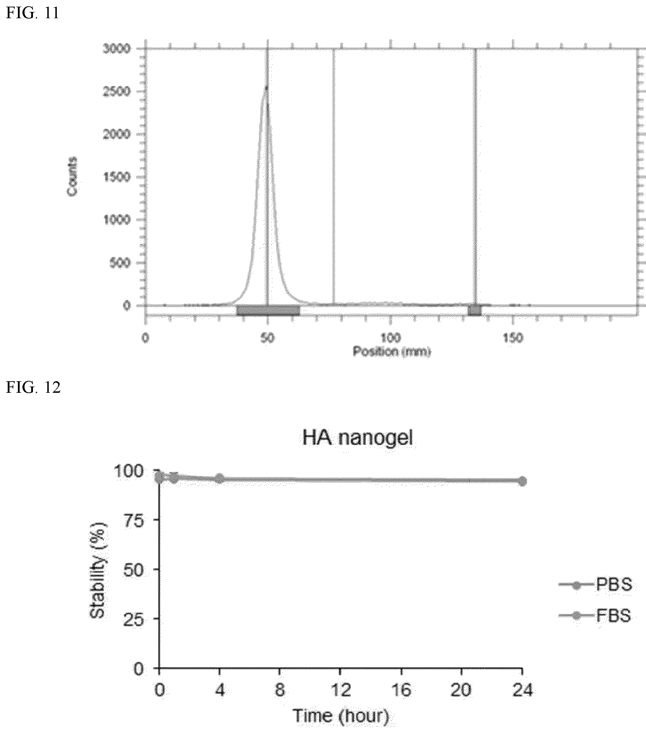

As a result, as confirmed in FIG. 11, it was confirmed that the radiochemical purity was very high as almost 100% and the excellent labeling reaction with Cu-64 was performed.

In the case of the stability of radiolabeled nanoparticles, the experiment was performed by checking radiolysis, and the stability of the nanoparticles was confirmed by time-dependant radio-TLC analysis using phosphate buffer saline (PBS) and fetal bovine serum (FBS).

As a result, like the result of FIG. 12, it was confirmed that high stability was shown up to 1 hour and 4 hours, and even in a result for 24 hours, excellent stability of 95% or more was shown in the same manner, and thus, it was expected that the stability may be sufficiently maintained even in a mouse.

### (2) In vivo distribution verifying experiment in normal mouse

An *in vivo* distribution verifying experiment for a normal mouse was performed by using the hyaluronic acid (HA) nanogels labeled with Cu-64 and a study to verify distribution for organs and *in vitro* release paths was conducted.

Thereafter, since the synthesized nanoparticles will be used for development and research of a nanogel-based tumor contrast agent, the experiment was planned with a time when the nanogels sufficiently targeted the tumor, and accordingly, the nanogels were injected and then experimented by a method of verifying a distribution for each organ after 24 hours.

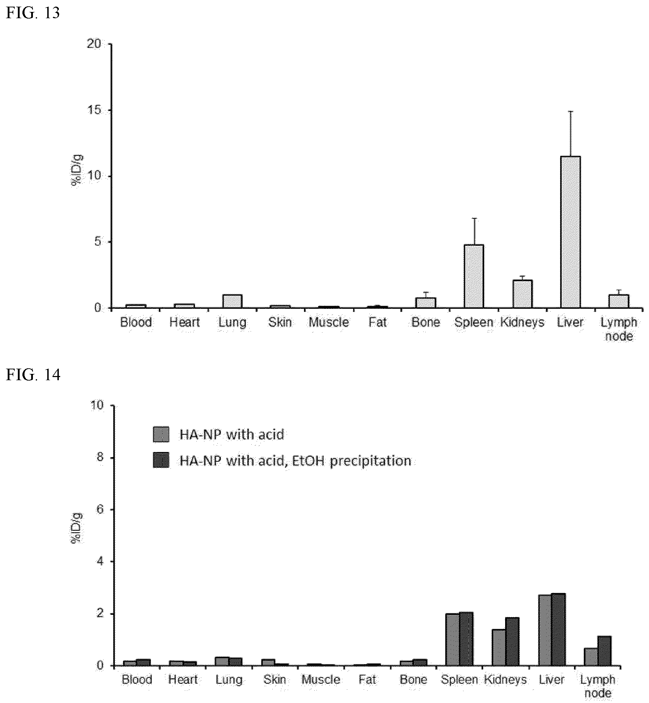

When confirming the results thereof, as illustrated in FIG. 13, in the case of the synthesized HA nanogels, the highest intake was shown in the liver.

Thereafter, in a process of synthesizing HA nanogels using an electron beam, a specific acid (HClO₄) was added to a beam-irradiated sample to change pH, and then an *in vivo* distribution verifying experiment was performed in a normal mouse (FIG. 14), and it was confirmed that the remaining degree in the body tended to be entirely much reduced as compared with the above result.

Further, even for HA nanogels obtained by a method of precipitating with an organic solvent other than a freeze-drying process of generally obtaining synthesized HA nanogels as a final sample, the *in vivo* distribution verifying experiment was performed, and as confirmed in FIG. 14, the intake levels for other organs were similar to each other.

Using nanoparticles made by the labeling reaction with Cu-64, the *in vivo* distribution verifying experiment was performed in a tumor model, and after 24 hours of injection, the distribution for each organ was confirmed and the intake level for the tumor was confirmed.

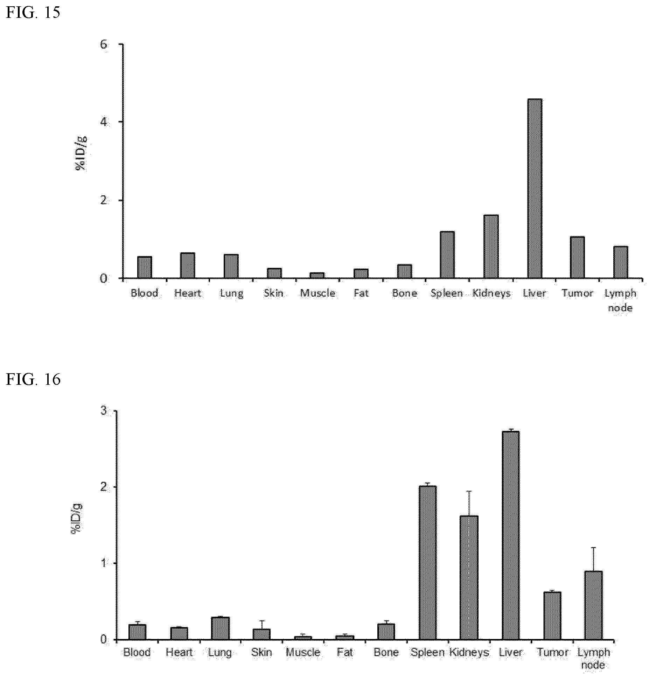

First, when a tumor model was prepared using colon tumor cells (CT26) and tumors grew in an appropriate size of 6 to 8 mm, the *in vivo* distribution verifying experiment was performed in the tumor model. As a result, as confirmed in FIG. 15, the highest intake of 4.5 %ID/g was shown for the liver and next, the intake for kidneys (1.6 %ID/g) and spleen (1.2 %ID/g) was high. However, even in the tumor, high intake was shown as the intake of 1.0 %ID/g as compared with other organs, and it was confirmed that a tumor to muscle ratio was 8.4 times and a tumor to blood ratio was 1.9 times to excellently diagnose the tumor.

In addition, the tumor model was prepared using skin cancer cells (melanoma/B16f10) and the *in vivo* distribution verifying experiment was performed after 24 hours when the tumor grew at an appropriate size. As confirmed in FIG. 16, high intake was shown in the liver, kidneys and spleen, but it was confirmed that the nanogels were released *in vitro* through the liver and kidneys, and the tendency to be released through the liver other than the kidneys was higher, and it was confirmed that the high intake was shown even in the spleen due to characteristic of the nanoparticles. Next, in tumor (0.62 %ID/g) and lymph node (0.89 %ID/g), it was confirmed that high intake was shown, and it was confirmed that the tumor to muscle ratio was 14.2 times and the tumor to blood ratio was 3.2 times to excellently diagnose the tumor.

A nuclear medicine imaging study for a tumor model was conducted by using hyaluronic acid nanogels labeled with radioisotope Cu-64, and the intake of detailed organs and the distribution and release for organs which have not been confirmed in the *in vivo* distribution verifying experiment performed above, and the target ability for tumor were confirmed.

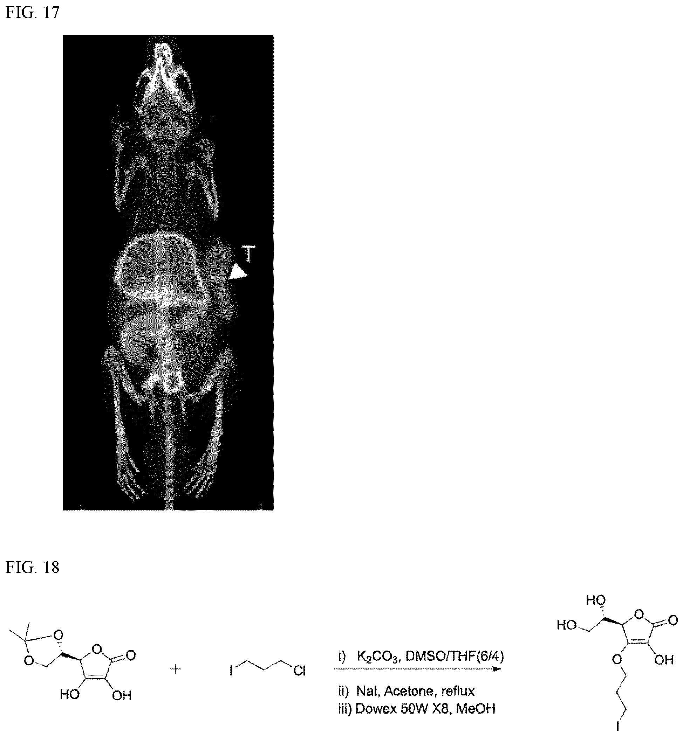

In the nuclear medicine imaging experiment, colon tumor cells (CT26) were used. As confirmed in FIG. 17, in a 5-hour PET image for hyaluronic acid nanogels, it can be seen that the highest signal was shown in the liver in the same manner as the *in vivo* distribution verifying experiment, and next, it was confirmed that the high intake was performed in order of colon and tumor.

### Example 3: Synthesis of hyaluronic acid nanoparticles bound with vitamin C and evaluation of efficacy thereof

### (1) Synthesis of hyaluronic acid nanoparticles bound with ascorbic acid

### 1) Synthesis of ascorbic acid derivative

As a method for effectively binding hyaluronic acid and ascorbic acid, a strategy to synthesize an ascorbic acid derivative by an ester bond to a carboxylic group of hyaluronic acid was established.

An alkylation condition (1-chloro-3-iodopropane, NaHCO₃, DMSO) capable of selectively binding to a hydroxyl group at position 3 of ascorbic acid protected in an acetal form was found and a protective group was removed to synthesize an ascorbic acid derivative in a form capable of binding to hyaluronic acid (FIG. 18).

Specifically, an ascorbic acid derivative was synthesized by binding 1-chloro-3-iodopropane to an OH group at position 3 of ascorbic acid protected in an acetal form, substituting a chloro group (Cl) with an iodo group (I), and removing an acetal group as a protective group (FIG. 18).

### (2) Synthesis of hyaluronic acid nanoparticles bound with ascorbic acid

The prepared ascorbic acid derivative was to bind to the hyaluronic acid nanoparticles prepared in Example 1.

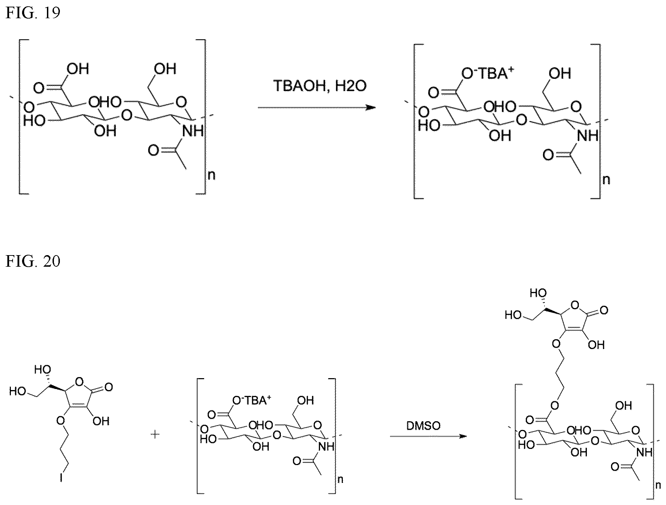

Since the hyaluronic acid nanoparticles having a carboxylic group were not dissolved in DMSO as a reaction solvent, a method of substituting to a tetrabutyl ammonium (TBA) group was used. Specifically, the hyaluronic acid nanoparticles containing the carboxylic group were dissolved in water and gradually added with TBAOH to adjust pH = 9 to 10, and then freeze-dried to prepare hyaluronic acid nanogels in TBA forms (FIG. 19).

Thereafter, a hyaluronic acid nanogel derivative bound with ascorbic acid in a powder form was obtained by stirring the prepared ascorbic acid derivative substituted with an iodo group with hyaluronic acid nanogels obtained in a TBA form under a DMSO solvent for about 12 hours and adding acetone to a reaction solution (FIG. 20).

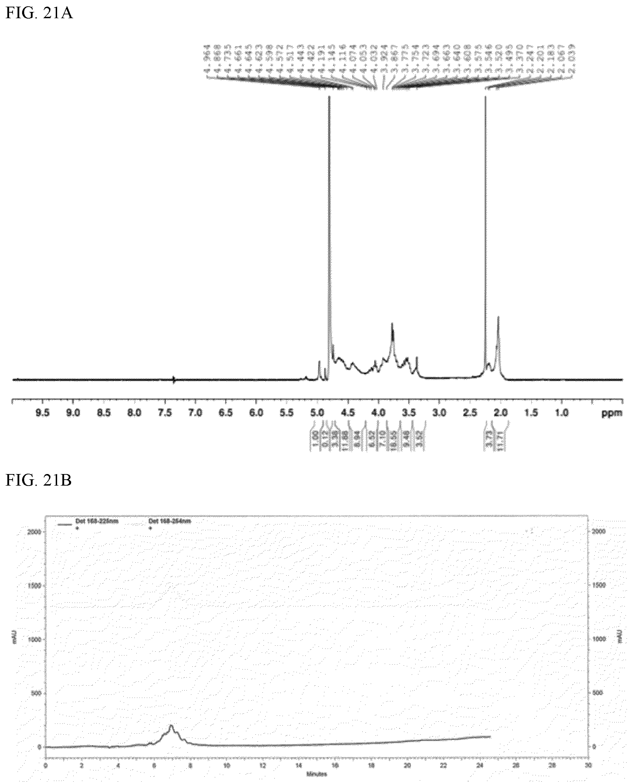

Whether the hyaluronic acid nanogel derivative bound with ascorbic acid was prepared was confirmed by NMR and HPLC chromatogram, which was shown in FIGS. 21A and 21B. As illustrated in FIG. 21, it was confirmed that as an ascorbic acid 1 proton peak in 4.9 ppm and a CH₂ peak of a propyl linker in 2.1 ppm were confirmed, an ester bond between ascorbic acid and hyaluronic acid was made (FIG. 21A), and a peak corresponding to ascorbic acid was confirmed even in HPLC chromatogram (FIG. 21B).

According to the NMR analysis result, it was confirmed that a ratio of ascorbic acid to carboxylic acid of a hyaluronic acid molecule was 2.5:1. That is, it was confirmed that one ascorbic acid per 2.5 carboxylic groups of the hyaluronic acid molecule was bound.

### (2) Evaluation of stability and efficacy of hyaluronic acid-ascorbic acid nanoparticles (HA-VitC)

Thermal stability evaluation was analyzed using HPLC after leaving ascorbic acid (AA, 100 µM) and the prepared hyaluronic acid-ascorbic acid nanogels (HA-VitC, 0.5 mg/mL) aqueous solution at 50°C for 48 hours, respectively.

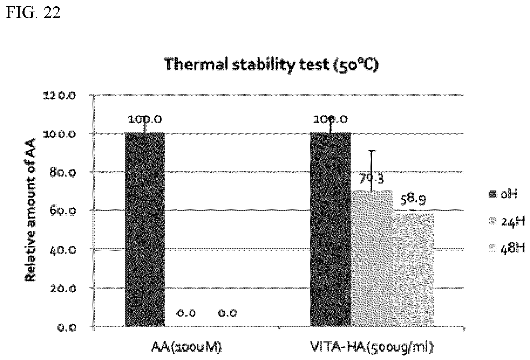

The result thereof was illustrated in FIG. 22.

As illustrated in FIG. 22, it was confirmed that the ascorbic acid (AA) was not oxidized over time, while the HA-VitC was stable as compared with the ascorbic acid (AA) (improved stability of 50% or more).

### (3) Evaluation of efficacy of whitening, antioxidation, and wrinkle improvement of hyaluronic acid-ascorbic acid nanoparticles (HA-VitC)

Whitening efficacy evaluation was measured by melanin contents assay and cellular tyrosinase activity assay after treating each experimental substance to a B16F10 mouse melanoma cell line and incubating the each experimental substance for 48 hours. As a control, ascorbic acid, ascorbic acid-2-glucoside (AA2G) and HA1 (Oligo-HA) used in Example 2 were used.

The substances used in the experiment were all left for 24 hours at - 20°C or 50°C and then used in the experiment.

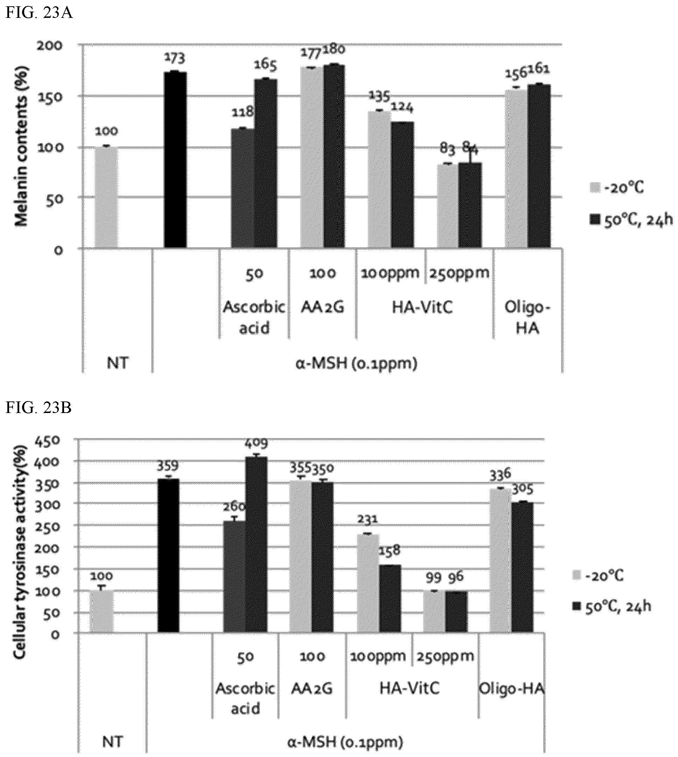

The result thereof was illustrated in FIG. 23.

As illustrated in FIGS. 23A and 23B, ascorbic acid, AA2G, HA-VitC, and oligo hyaluronic acid (Oligo-HA) were left for 24 hours at 50°C, respectively, and then as a result of comparing efficacy with a sample stored at - 20°C, in the ascorbic acid, the whitening efficacy disappeared during the storage of 50°C, but in the case of HA-VitC, it was confirmed that the melanin production and the tyrosinase activity were still strongly inhibited in a concentration-dependent manner.

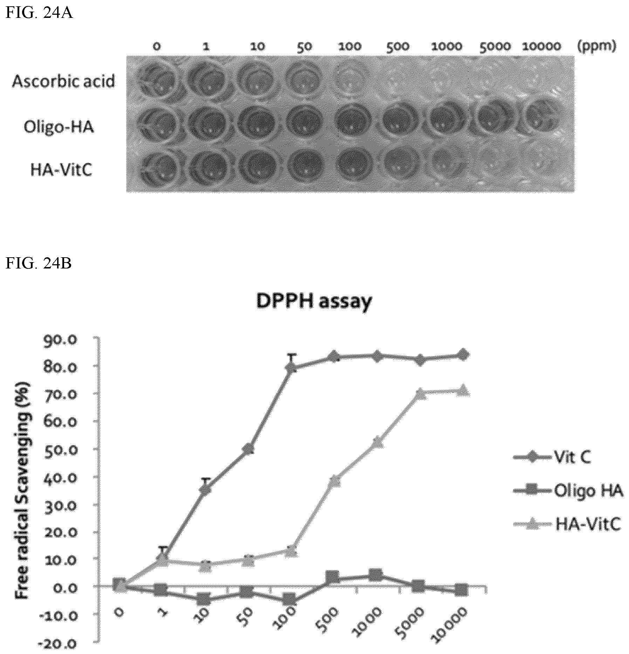

Then, the free radical scavenging ability of HA-VitC was evaluated using DPPH assay. Specifically, ascorbic acid, oligo-HA and HA-VitC were treated for each concentration shown in FIG. 24, and then the free radical scavenging ability was evaluated according to a conventionally known method (FIG. 24A), and quantified and shown in a graph (FIG. 24B).

Referring to FIGS. 24A and 24B, in the case of the hyaluronic acid (Oligo-HA), since there was no free radical scavenging ability, it was determined that the free radical scavenging ability of HA-VitC was shown by Vitamin C (Vit C) binding to the HA nanogels in HA-VitC.

Particularly, when considering that vitamin C is very vulnerable to oxidation after exposure to air and then quickly decomposed, in the case of HA-VitC according to the present disclosure, it is determined that utilization is very high by improving the storage stability of vitamin C and having the effect of Vit C as it is at the same time.

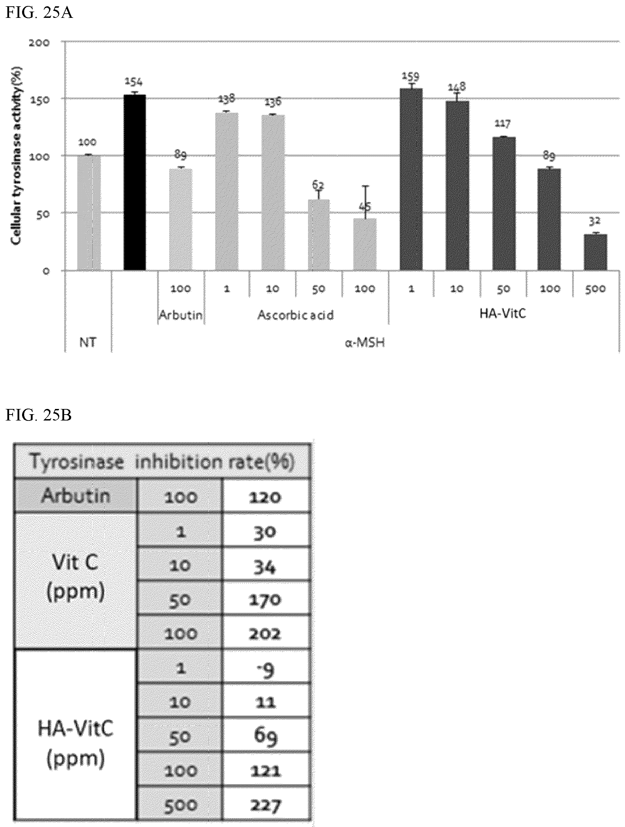

Next, the cellular tyrosinase inhibitory activity of HA-VitC was evaluated according to a known method:
Cell line: B16F10 mouse melanoma (Passage 10), 6 well plate, 4 x 10⁵ cells/well
Evaluation method: Cellular tyrosinase activity assay
Experimental substance treatment time: 48 hours
Experimental substance treatment concentration: See FIG. 25

The result thereof was illustrated in FIG. 25.

As illustrated in FIGS. 25A and 25B, it was confirmed that HA-VitC inhibited the cellular tyrosinase in a concentration-dependent manner.

Next, the fibroblast proliferation effect was evaluated according to a conventional known method.

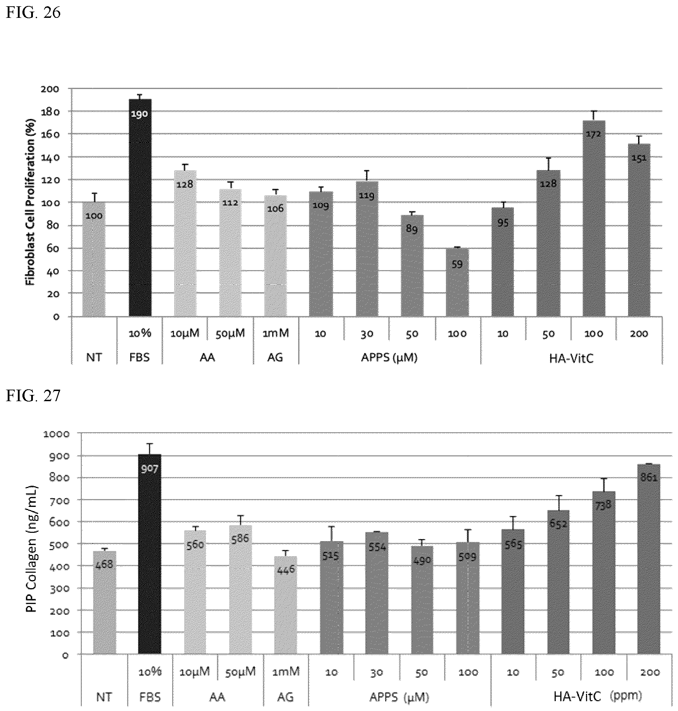

Briefly, experimental conditions are as follows, and a type of experimental substance and a treatment concentration of each substance are shown in FIG. 26:
Cell line: HDF-neo, Human primary dermal fibroblast-neonatal (passage 15), 24 well plate, 1 x 10⁴ cells/well
Evaluation method: WST-8 assay(Ez-cytox) and Procollagen ELISA (Takara #MK101)
Experimental substance treatment time: 3 day, 1day fasting, serum free condition

The result thereof was illustrated in FIG. 26.

As illustrated in FIG. 26, it was confirmed that in the case of ascorbic acid, AA2G (AG), and APPS, the fibroblast proliferation promoting efficacy was almost not shown, but in the case of HA-VitC, it was confirmed that the proliferation of fibroblasts was promoted in a concentration-dependent manner.

Next, the effect of HA-VitC on collagen biosynthesis was evaluated according to a conventional known method. Briefly, experimental conditions are as follows, and a type of experimental substance and a treatment concentration of each substance are shown in FIG. 27:
Cell line: HDF-neo, Human primary dermal fibroblast-neonatal (passage 11), 96 well plate, 3 x 10³ cells/well
Evaluation method: Procollagen ELISA (Takara #MK101)
Experimental substance treatment time: 3 day, 1day fasting, serum free condition

The result thereof was illustrated in FIG. 27.

As illustrated in FIG. 27, it was confirmed that the highest collagen biosynthesis was induced in a HA-VitC treating group.

### INDUSTRIAL APPLICABILITY

The present disclosure provides a method for preparing pure hyaluronic acid nanoparticles on an aqueous solution without a cross-linking agent and an organic solvent. The present disclosure may be very usefully used for developing a contrast agent for tumor diagnosis and a therapeutic agent of synthesized hyaluronic acid nanoparticles through the skin permeability confirmed by fluorescence labeling and the radioactive labeling by uniformly synthesizing various nano-meter sizes of hyaluronic acid nanoparticles reproducibly under an optimized electronic beam condition, and therefore, industrial applicability is very excellent.

## Claims

1. A method for preparing cross-linked hyaluronic acid nanoparticles, comprising:
irradiating an electron beam to a hyaluronic acid aqueous solution, thereby forming intermolecular or intramolecular cross-linked hyaluronic acid nanoparticles without a separate cross-linking agent in the hyaluronic acid aqueous solution;
adjusting the pH of the hyaluronic acid aqueous solution, thereby controlling the size of the hyaluronic acid nanoparticles; and
wherein the pH of the hyaluronic acid aqueous solution is 1 to 4.

2. The method of claim 1, wherein HClO₄ is additionally included in the hyaluronic acid aqueous solution; or
wherein the irradiating dose of the electron beam is changed to control the size of the hyaluronic acid nanoparticle; or
wherein the irradiating dose of the electron beam is increased to reduce the size of the hyaluronic acid nanoparticle.

3. The method of claim 1, wherein the hyaluronic acid aqueous solution has a concentration of 0.05 to 7% (w/v).

4. The method of claim 1, wherein the electron beam is irradiated at an irradiation dose of 0.5 to 300 kGy.

5. The method of claim 1, wherein the hyaluronic acid nanoparticle has a diameter of 100 nm or less as measured by DLS and/or TEM.

6. A conjugate of ascorbic acid and hyaluronic acid nanogels, wherein the hyaluronic acid nanogels are prepared according to the method of any one of claims 1 to 5,
wherein preferably the conjugate has an improved storage stability of ascorbic acid.

7. The conjugate of claim 6, wherein the conjugate is represented by the following Chemical Formula 2: 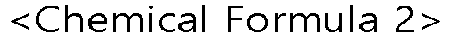 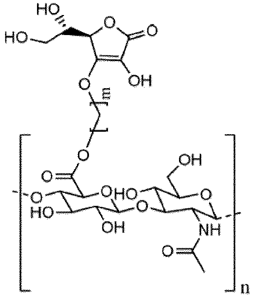 Wherein m is 1 to 5

8. The conjugate of claim 6, wherein the conjugate is prepared by the following Reaction Formulas 1 to 3: 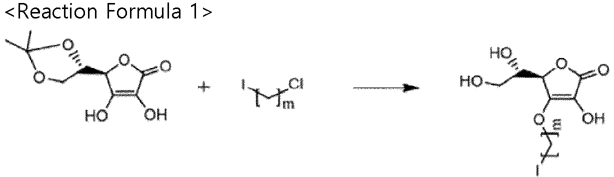 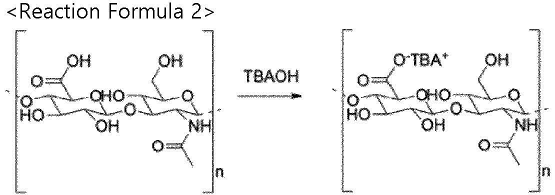 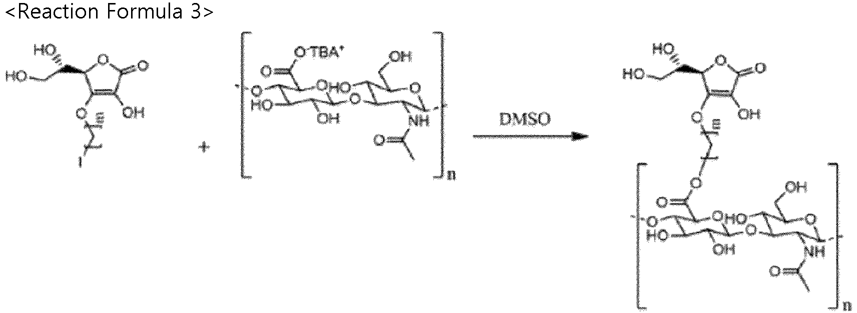 wherein m is 1 to 5.

9. A cosmetic composition for antioxidation, skin whitening, wrinkle improvement or skin anti-aging comprising the conjugate according to any one of claim 6 to 8 as an active ingredient.

10. A food composition for antioxidation, skin whitening, wrinkle improvement or skin anti-aging comprising the conjugate according to any one of claims 6 to 9 as an active ingredient.

## Patentansprüche

1. Verfahren zur Herstellung von vernetzten Hyaluronsäure-Nanopartikeln, umfassend:
Bestrahlung einer wässrigen Hyaluronsäurelösung mit einem Elektronenstrahl, wodurch intermolekular oder intramolekular vernetzte Hyaluronsäure-Nanopartikel ohne ein separates Vernetzungsmittel in der wässrigen Hyaluronsäurelösung gebildet werden;
Einstellen des pH-Werts der wässrigen Hyaluronsäurelösung, wodurch die Größe der Hyaluronsäure-Nanopartikel gesteuert wird; und
wobei der pH-Wert der wässrigen Hyaluronsäurelösung 1 bis 4 beträgt.

2. Verfahren nach Anspruch 1, wobei zusätzlich HClO₄ in der wässrigen Hyaluronsäurelösung enthalten ist; oder
wobei die Bestrahlungsdosis des Elektronenstrahls verändert wird, um die Größe der Hyaluronsäure-Nanopartikel zu steuern; oder
wobei die Bestrahlungsdosis des Elektronenstrahls erhöht wird, um die Größe der Hyaluronsäure-Nanopartikel zu verringern.

3. Verfahren nach Anspruch 1, wobei die wässrige Hyaluronsäurelösung eine Konzentration von 0,05 bis 7 % (w/v) aufweist.

4. Verfahren nach Anspruch 1, wobei mit einem Elektronenstrahl mit einer Bestrahlungsdosis von 0,5 bis 300 kGy bestrahlt wird.

5. Verfahren nach Anspruch 1, wobei die Hyaluronsäure-Nanopartikel einen Durchmesser von 100 nm oder weniger aufweisen, gemessen mittels DLS und/oder TEM.

6. Ein Konjugat aus Ascorbinsäure und Hyaluronsäure-Nanogelen, wobei die Hyaluronsäure-Nanogele gemäß dem Verfahren nach einem der Ansprüche 1 bis 5 hergestellt werden,
wobei das Konjugat vorzugsweise eine verbesserte Lagerstabilität von Ascorbinsäure aufweist.

7. Das Konjugat nach Anspruch 6, wobei das Konjugat durch die folgende chemische Formel 2 dargestellt wird: 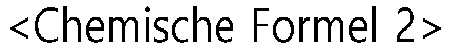 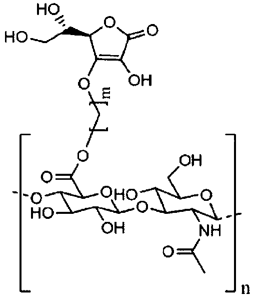 wobei m 1 bis 5 ist

8. Das Konjugat nach Anspruch 6, wobei das Konjugat durch die folgenden Reaktionsformeln 1 bis 3 hergestellt wird: 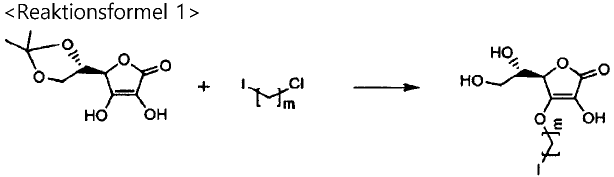 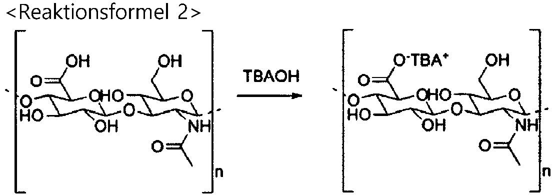 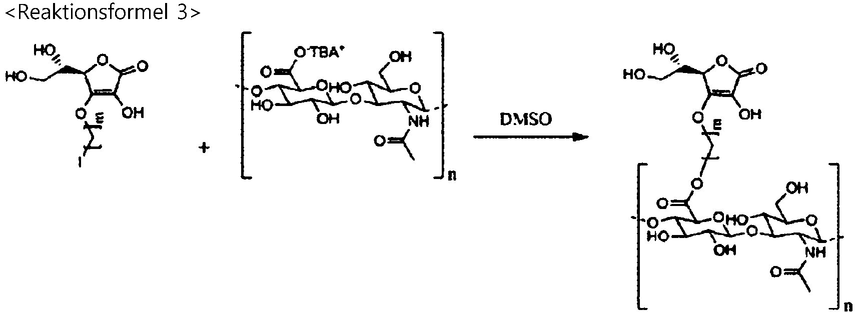 wobei m 1 bis 5 ist.

9. Kosmetische Zusammensetzung zur Antioxidation, Hautaufhellung, Faltenverbesserung oder Hautalterungsbekämpfung, die das Konjugat gemäß einem der Ansprüche 6 bis 8 als Wirkstoff enthält.

10. Eine Lebensmittelzusammensetzung zur Antioxidation, Hautaufhellung, Faltenverbesserung oder Hautalterungsbekämpfung, die das Konjugat gemäß einem der Ansprüche 6 bis 9 als Wirkstoff enthält.

## Revendications

1. Procédé de préparation de nanoparticules d'acide hyaluronique réticulées, comprenant:
l'irradiation d'un faisceau d'électrons sur une solution aqueuse d'acide hyaluronique, formant ainsi des nanoparticules d'acide hyaluronique réticulées de manière intermoléculaire ou intramoléculaire sans agent de réticulation séparé dans la solution aqueuse d'acide hyaluronique;
l'ajustement du pH de la solution aqueuse d'acide hyaluronique, contrôlant ainsi la taille des nanoparticules d'acide hyaluronique; et
dans lequel le pH de la solution aqueuse d'acide hyaluronique est de 1 à 4.

2. Procédé selon la revendication 1, dans lequel HClO₄ est en outre inclus dans la solution aqueuse d'acide hyaluronique; ou
dans lequel la dose d'irradiation du faisceau d'électrons est modifiée pour contrôler la taille de la nanoparticule d'acide hyaluronique; ou
dans lequel la dose d'irradiation du faisceau d'électrons est augmentée pour réduire la taille de la nanoparticule d'acide hyaluronique.

3. Procédé selon la revendication 1, dans lequel la solution aqueuse d'acide hyaluronique a une concentration de 0,05 à 7 % (p/v).

4. Procédé selon la revendication 1, dans lequel le faisceau d'électrons est irradié à une dose d'irradiation de 0,5 à 300 kGy.

5. Procédé selon la revendication 1, dans lequel la nanoparticule d'acide hyaluronique a un diamètre de 100 nm ou moins tel que mesuré par DLS et/ou TEM.

6. Conjugué d'acide ascorbique et de nanogels d'acide hyaluronique, dans lequel les nanogels d'acide hyaluronique sont préparés selon le procédé de l'une quelconque des revendications 1 à 5,
dans lequel de préférence le conjugué a une stabilité au stockage améliorée de l'acide ascorbique.

7. Conjugué selon la revendication 6, dans lequel le conjugué est représenté par la formule chimique 2 suivante: 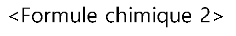 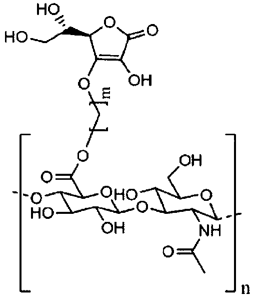 où m vaut de 1 à 5.

8. Conjugué selon la revendication 6, dans lequel le conjugué est préparé par les formules réactionnelles 1 à 3 suivantes: 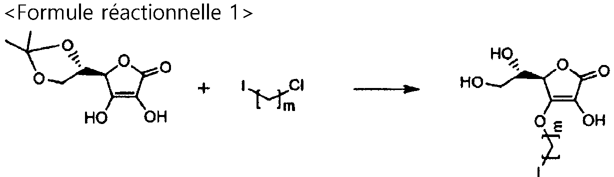 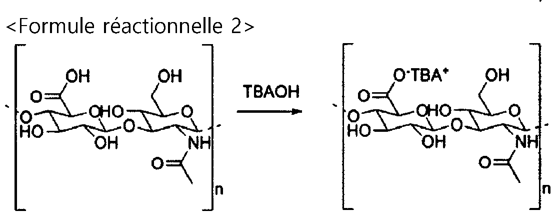 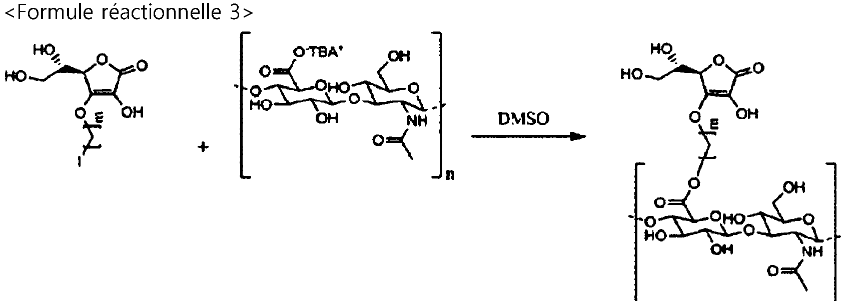 où m vaut de 1 à 5.

9. Composition cosmétique pour l'antioxydation, le blanchiment de la peau, l'amélioration des rides ou l'antivieillissement de la peau comprenant le conjugué selon l'une quelconque des revendications 6 à 8 en tant qu'ingrédient actif.

10. Composition alimentaire pour l'antioxydation, le blanchiment de la peau, l'amélioration des rides ou l'antivieillissement de la peau comprenant le conjugué selon l'une quelconque des revendications 6 à 9 en tant qu'ingrédient actif.
